# EUROPEAN PATENT APPLICATION

(11) **EP 4 722 386 A1**
(43) Date of publication of application: **08.04.2026**
(21) Application number: 23937243.6
(22) Date of filing: 30.10.2023
(51) Int. Cl.: C12Q 1/6874, C12Q 1/6811, C12Q 1/6888, C40B 40/06

(54) **MULTIPLE SINGLE NUCLEOTIDE POLYMORPHISM-BASED PORCINE 50K LIQUID CHIP, AND USE THEREOF**

(30) Priority: 17.05.2023 CN 202310552851
(71) Applicant: China Agricultural University, Beijing 100193 (CN)
(72) Inventor: DING, Xiangdong, Beijing 100193 (CN); LIU, Huatao, Beijing 100193 (CN); DOU, Hehe, Beijing 100193 (CN); ZHANG, Zipeng, Beijing 100193 (CN); ZHANG, Qin, Beijing 100193 (CN); WANG, Chuduan, Beijing 100193 (CN)
(74) Representative: Maiwald GmbH
(86) International application number: PCT/CN2023/127964
(87) International publication number: WO 2024/234568

(57) **Abstract**

This invention relates to the field of genetic molecular breeding, specifically to a pig 50K liquid-phase chip based on multiple single nucleotide-polymorphism and its application. The probe design of the chip in this invention takes into account the distribution of captured SNP loci across the genome, the polymorphism of the loci, the quality of mSNP markers, and other issues, effectively avoiding problems such as uneven marker density and poor polymorphism. It also considers the quality of mSNP markers and the issue of linkage disequilibrium among mSNP markers. While adhering to the basic principles of liquid-phase chip design, genomic regions with moderate linkage disequilibrium between markers were selected, generating more SNP markers with high genotyping quality and moderate linkage disequilibrium within the probe region with the target loci. The mSNP liquid-phase chip of this invention expands the detectable number of SNPs to 1.5-2 times that of the target loci, addressing the issue of the relatively small number of high-quality mSNP markers in traditional liquid-phase chips without increasing costs.

## Description

### Technical Field

This invention relates to the field of genetic molecular breeding, specifically to a pig 50K liquid-phase chip based on multiple single nucleotide polymorphism technologies and its application, more specifically a pig 50K mSNP liquid-phase chip.

### Background Technology

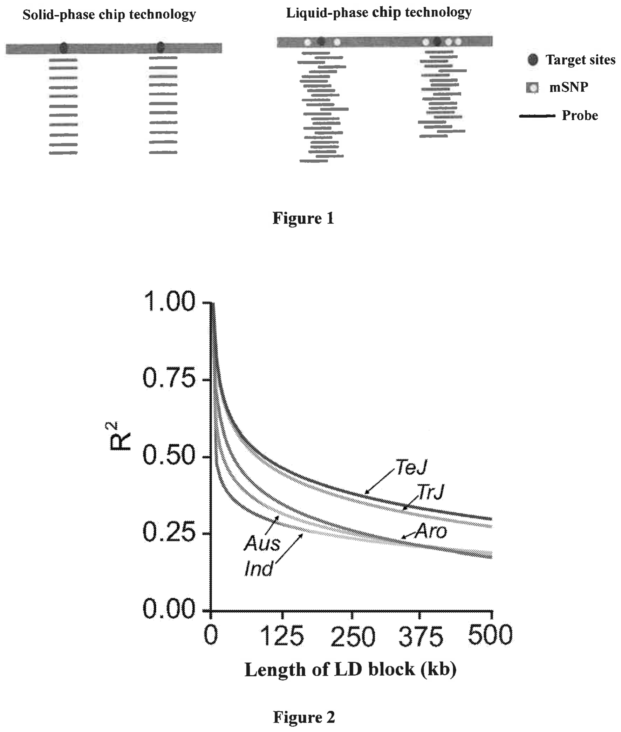

Single nucleotide polymorphism (SNP) is characterized by its large number, wide distribution across the genome, ease of large-scale rapid screening, and genotyping, making it the best molecular marker available today. The rapid development of molecular detection technology has given rise to SNP chips capable of high-throughput genotyping. At present, the mainstream SNP chips on the market are mainly developed based on solid-phase technology, with relatively high detection accuracy. As shown in Figure 1, solid-phase chips primarily perform multiple detections on target sites to ensure the accuracy of genotyping. However, solid-phase chips have disadvantages such as poor flexibility, strict sample size requirements (must be a multiple of 12 or 24), and high customization costs, limiting their large-scale use in practical breeding.

Unlike solid-phase chips, liquid-phase chips based on genotyping by target sequencing (GBTS) technology have advantages such as easy addition or removal of markers and no sample size requirements. Figure 1 shows that GBTS technology mainly performs multiple sequencing of the target site and its upstream and downstream regions to ensure the quality of target site genotyping. Unlike solid-phase chips, liquid-phase chips can also genotype polymorphic sites upstream and downstream of the target site, known as multiple single nucleotide polymorphism clusters (mSNPs or multiple dispersed nucleotide polymorphisms, MNPs). The mSNPs centered on target sites are tightly linked and are in a state of high linkage disequilibrium. In many species, the genotypes of mSNPs upstream and downstream of the target site are considered consistent with the target site and do not provide additional information. As shown in Figure 2, the linkage disequilibrium (r2) within a 200bp fragment of multiple rice varieties is 1, indicating that the SNP genotypes within these fragments are linked and identical. Even if there are multiple mSNPs, the information provided is the same as that of a single target site. Therefore, although liquid-phase chips can detect mSNPs exceeding the number of target sites, the mSNP information upstream and downstream of the target site is rarely used, and genetic analysis and molecular breeding mainly focus on the target site.

Although high linkage disequilibrium between closely linked markers exists in animal genomes, due to the high diversity of animal genomes and high average heterozygosity of markers, there are many genomic regions where the degree of linkage disequilibrium between markers is moderate. For liquid-phase chips, markers upstream and downstream of the target site can provide additional information. Therefore, multiple single nucleotide polymorphism technology can clearly increase the number of effective mSNP markers without increasing the number of target site markers, thereby enhancing the information content of liquid-phase chips and fully utilizing the characteristics of GBTS technology; this is not attainable with solid-phase microarray technologies.

Although multiple single nucleotide polymorphism technology increases mSNP markers, there are many challenges in utilizing this marker information. Directly using mSNPs as single markers often introduces noise due to high linkage disequilibrium between markers, reducing the effectiveness of genetic analysis. Haplotype analysis can simultaneously utilize information from multiple SNP markers, improving the effectiveness of genetic analysis. However, many studies have shown that if the marker spacing is too large, the low linkage disequilibrium between markers can result in numerous haplotypes, which not only fails to increase the power of genetic analysis but also adds complexity to the analysis and increases computation time. Currently, the mainstream 50K SNP chips for pigs, such as the SNP chip Porcine GGP 50K designed by Neogen Corporation (containing 50,697 SNP markers), have an average marker spacing of 40 kb and an average linkage disequilibrium of 0.2. Theoretical research and breeding practices have shown that haplotype analysis with a fixed segment length or a fixed number of SNPs cannot improve the accuracy of genomic selection. For liquid-phase chips, although the average spacing and linkage disequilibrium level of target sites are similar to those of the SNP chip Porcine GGP 50 K, mSNP markers upstream and downstream of the target site have much smaller spacing, greatly enhancing the degree of linkage disequilibrium between markers. Treating them as a block for haplotype analysis can significantly improve the accuracy of genomic selection and the effectiveness of genetic analysis.

### Summary of the Invention

This invention has developed a 50K liquid-phase chip for pigs and also provides methods for chip target site development and analysis. Using the chip and analysis strategy designed by this invention maximizes the use of mSNP marker information upstream and downstream of the target site, improving the efficiency of genetic analysis and molecular breeding in pigs.

The first aspect of the present invention provides a method for 50K mSNP marker selection and probe preparation for a 10K liquid phase chip used for multiple single nucleotide-polymorphism, which utilizes whole-genome sequencing data of pig breeds to mine and screen target SNP loci, then designs and optimizes probes for the target SNP loci, ultimately resulting in the determination of the probes. The method includes the following steps:
Step 1, determining target site SNPs: Based on whole-genome sequencing data of three pig breeds-Duroc, Large White, and Landrace-and aligning them to the whole-genome sequencing data of pig breeds, genomic regions with moderate linkage disequilibrium between markers were selected, and targeted capture sites were screened, i.e., target site SNPs.

Step 2, designing probes based on the determined target site SNPs: Utilize the multiple single nucleotide polymorphism technologies to design 1-4 probes, each 110 bp in length, centered on each target SNP. Each probe covers the target SNP, with a total probe coverage of 165 bp around the target SNP. The principles for probe design are: 1) Select probes with a content between 30% and 80%; 2) Choose regions with a number of homologous areas ≤ 5; 3) Select probe areas that do not contain SSR, N regions.

Step 3, selecting and optimizing probes containing high-quality mSNPs: Probes are hybridized and sequenced, and the genotyping quality of mSNPs, including target sites, is detected. Set a missing rate of NA<0.1, a minimum allele frequency (MAF) ≥0.05, and heterozygosity (Het) <0.5 as standards to screen mSNPs, removing those that do not meet the standards. If the probe does not meet the genotype quality control requirements of mSNPs, delete the probe and the corresponding target site SNP, redesign new probes according to Steps 1 and 2, and continue to test and optimize the probes as per this step. The mSNPs that meet the quality inspection requirements are finally used as the target sites of the 50K mSNP liquid-phase chip.

Specifically, the whole-genome sequencing data of pig breeds is the 11.1 reference genome; the principles for screening target site SNPs are: ① Uniform distribution across chromosomes, with denser distribution at both ends of the chromosomes; ② Polymorphism considers MAF > 0.35 in Duroc, Landrace, and Large White pigs; ③ Average linkage disequilibrium (r2) with upstream and downstream SNP markers less than 0.85; ④ Comparison with the QTLdb database, aiming for SNP markers to be located in QTL regions related to economic traits; ⑤overlap with some loci on the known 50K chip for pigs.

More specifically, the known pig 50K chip referred to is a 50K SNP liquid-phase microarray, the GGP50K from the American company Neogen, or the Zhongxin No.1. The second aspect of the present invention provides a pig 50K mSNP liquid-phase chip based on multiple single nucleotide polymorphism technologies, which is prepared based on the probes obtained by the method described.

The preparation steps are as follows: after synthesizing the probes, mix them in equimolar amounts, adjust the concentration to 1-5 pmol/mL in a buffer solution, and then prepare the probe hybridization solution to obtain the final product.

Specifically, the buffer is a mixture of EDTA and Tris-HCl.

More specifically, the method further includes using a Pooled, barcoded library, GenoBaits Block I, and GenoBaits Block II for ILM/MGI to prepare the probe hybridization solution with the following components:

| Component name | Quantity |
|---|---|
| Pooled, barcoded library | 0.6 µL |
| GenoBaits Block I | 5 µL |
| GenoBaits Block II forILM/MGI | 2 µL |
| The said probe | 300 ng |

Preferably, the probe hybridization solution is concentrated to dryness using a vacuum concentrator at a temperature of ≤ 60°C.

The third aspect of the present invention provides the application of the said pig 50K mSNP liquid-phase chip, specifically a method for detecting pig individual genotypes. The said method includes the following steps: obtaining samples from the pigs to be tested and extracting genomic DNA; constructing pig cDNA libraries; hybridizing and sequencing the constructed libraries with the pig 50K mSNP liquid-phase chip; performing mSNP genotyping according to the sequencing data operation process, and determining the genotypes of all liquid-phase chip marker loci for each individual.

Preferably, the mSNP genotype analysis method is as follows:
Step 1: After determining the genotypes of all mSNP markers for the individual liquid-phase chip, perform quality control on the mSNP genotypes; quality control is carried out in the following order:
1) Filter out multi-allelic variants;
2) Remove sex chromosomes and loci with unknown positions;
3) Remove SNPs with a call rate below 90%;
4) Remove SNPs with a minor allele frequency (MAF) below 0.05;
5) Remove individuals with a call rate below 90%.

Step 2: Using the target site SNP as the core, define a 200bp upstream and downstream region as a haplotype block, dividing the genome into 52,000 haplotype blocks, each with at least one mSNP marker, with varying numbers.

Step 3: For each haplotype block, infer haplotypes, determine haplotype alleles, and construct haplotype genotypes or diplotypes for each haplotype block in the tested sample, thereby constructing diplotype vectors for all haplotype blocks in the tested sample, similar to genotype vectors for all mSNP markers.

Step 4: Based on the diplotype vectors of all samples, apply genetic analysis or molecular breeding methods, with each haplotype block treated as a marker, and haplotypes within the block as alleles and diplotypes as genotypes.

Compared to existing technology, the method provided by the present invention for developing the pig 50K mSNP liquid-phase chip has the following beneficial effects: The present invention provides a high-throughput SNP50K probe for pigs based on targeted capture sequencing for genotyping. The probe design considers the distribution of captured SNP loci across the genome, locus polymorphism, mSNP marker quality, and other issues. In the Duroc, Landrace, and Large White pig populations, the target loci MAF requirement is greater than 0.35, effectively avoiding issues such as uneven marker density and poor polymorphism.

Compared to existing liquid-phase chips, the present invention considers the quality of mSNP markers and the issue of linkage disequilibrium among mSNP markers. While adhering to the basic principles of liquid-phase chip design, genomic regions with moderate linkage disequilibrium between markers were selected, generating more SNP markers with high genotyping quality and moderate linkage disequilibrium within the probe region with the target loci. These markers are collectively referred to as mSNP. The mSNP liquid-phase chip can generate multiple SNP markers at a single amplification site (target site), expanding the number of detectable SNPs to 1.5-2 times that of the loci. This solves the problem of the relatively small number of high-quality mSNP markers in traditional liquid-phase chips without increasing costs. Additionally, the present invention provides an effective method for utilizing mSNP markers. Compared to traditional single-marker analysis and haplotype analysis methods, the present invention provides a haplotype block method centered on the target site that includes its upstream and downstream mSNPs. This method fully utilizes the linkage disequilibrium information of SNP markers within the probe, improving the efficiency of genetic analysis and molecular breeding. It avoids the noise caused by mSNPs within the probe in traditional single-marker analysis, which can result in excessive bias, as well as the low efficiency of fixed SNP number or fragment length haplotype analysis methods.

Therefore, based on the developed pig 50K liquid-phase chip and the characteristics of the pig genome, the present invention has developed the pig 50K mSNP liquid-phase chip using multiple single nucleotide polymorphism technologies. Compared to existing liquid-phase chips, the present invention increases the number of effective mSNP markers without increasing costs, resulting in greater information content. Concurrently, mSNPs can be leveraged in conjunction with haplotype and other analytical techniques to enhance the efficacy of genetic analysis and molecular breeding endeavors. Furthermore, the liquid-phase chip of the present invention can achieve DNA hybridization capture time within 1 hour, significantly shortening the time required to obtain genotypes compared to the overnight hybridization capture process that takes more than 16 hours. The entire process of library construction and capture can be completed within one day.

### Description of the Drawings

Figure 1 is a schematic diagram of solid-phase chip and liquid-phase chip technologies.
Figure 2 illustrates the decay of linkage disequilibrium (LD) among five subspecies of rice (Yan et al., 2020). The five subspecies are Australian rice (Aus), Fragrant rice (Aro), Tropical rice, Japonica rice (TrJ), Temperate Japonica rice (TeJ), and the wild rice subspecies (Oru), as well as Indica rice. The markers among these five subspecies of rice indicate that the R2 value for intervals of several hundred base pairs is 1, which signifies complete LD.
Figure 3 shows the number (A) and density distribution (B) of target site SNPs on each chromosome in this invention.
Figure 4 shows the distribution of target sites (in red) and mSNPs (in blue) on each chromosome for the 50K mSNP liquid-phase chip in Farm 1.
Figure 5 shows the density distribution of target sites (A) and all mSNPs (B) on each chromosome for the 50K mSNP liquid-phase chip in Farm 1.
Figure 6 shows the distribution (A) and decay of linkage disequilibrium (B) of all loci after quality control for the 50K mSNP liquid-phase chip.
Figure 7 shows the process of constructing a haplotype matrix.
Figure 8 shows the distribution of the number of mSNPs for each probe (A) and the average linkage disequilibrium level between adjacent markers under single-marker and different haplotype block division schemes (B).

### Specific Method of Embodiment

Below, the specific embodiments of the invention are described in further detail in conjunction with the accompanying drawings and examples. The following examples are intended to illustrate the invention but are not intended to limit its scope.

### Example 1: Screening Method for SNP Markers and Probe Preparation Required for the Invention

### Step 1: Target SNP marker selection

In conjunction with the 50K liquid-phase chip invented by the applicant, based on the whole-genome sequencing data of the Duroc, Large White, and Landrace pig breeds, comparison is made to the pig 11.1 reference genome. Genomic regions with a moderate degree of linkage disequilibrium between markers are selected, and targeted capture sites, i.e., target site SNPs, are screened. The principles for screening target site SNPs are: ① Uniform distribution across chromosomes, with denser distribution at both ends of the chromosomes; ② Polymorphism mainly considers MAF > 0.35 in Duroc, Landrace, and Large White pigs; ③ The average linkage disequilibrium level (r2) with upstream and downstream SNP markers is less than 0.85; ④ Comparing with the QTLdb database, aiming for SNP markers to be located in QTL regions related to economic traits; ⑤There is partial overlap with some loci of the 50K chips currently on the market (50KSNP liquid-phase chip, Neogen's GGP50K in the United States, and Zhongxin No.1), especially including the important candidate loci for growth, reproduction, feed conversion, and body size traits that the applicant had previously developed for the 50KSNP liquid-phase chip (CN202110359470.7 - A 50K liquid-phase chip for pigs based on targeted capture sequencing and its application), ensuring chip compatibility.

### Step 2: Design probes based on the target site SNPs

Probes are optimized and designed using multiple single nucleotide-polymorphism. For each target SNP, 1-4 probes of 110bp length are designed, with each probe covering the target SNP. The total coverage of probes centered on the target SNP is 165bp in length. The principles of probe design are: 1) Select probes with content between 30%-80%; 2) Select regions with a homology number ≤5; 3) Exclude regions containing SSR or N regions in the probe.

### Step 3: Through multiple sequencing and hybridization of probes, select probes containing high-quality mSNPs, and optimize the probes.

Probes are sequenced and hybridized to detect the genotyping quality of target sites and mSNPs. Set a missing rate of NA<0.1, a minimum allele frequency (MAF) ≥0.05, and heterozygosity (Het) <0.5 as standards to screen mSNPs, with removal of those that do not meet the standards. If the probe does not meet the genotype quality control requirements of mSNPs, delete the probe and the corresponding target site SNP, redesign new probes according to Steps 1 and 2, and continue to test and optimize the probes as per this step. The mSNPs that meet the quality inspection requirements are finally used as 50K mSNP liquid-phase chip loci.

The method described in step three is designed to ensure the optimal quantity and quality of mSNP markers (including target SNPs) under the same target SNP probe. This invention includes 52,000 target sites and ultimately 80,631 high-quality probes. Compared to the already developed pig 50K liquid-phase chip, 3,385 additional probes containing 5-7 mSNPs are added, mainly concentrated in intergenic and intronic regions. The probes containing 5-7 mSNPs have an average interval of 550Kb. Table 1 lists some of the probe information containing 5-7 mSNPs on chromosome 18.

**Table 1: Probe information containing 5-7 mSNP markers (chromosome 18)**

| Probe Numb er | Probe Sequence | Target Site Position | Probe Start Position | Probe End Position | Numbe r of mSNP Marker s in the Probe |
|---|---|---|---|---|---|
| 18_14 32455 | TATTTGCAGAGTCCCAGCTGCCCCATCTAGTGATCTCTGCATG GAGCCACCCGGCAGGCCCTGGTAACCAGGTCGAGACACATTT TCCTTTGTCCCATTGTCCAAACTGG | 1432455 | 1432373 | 1432482 | 5 |
| 18_21 19817 | TCCCAAGTCCCTGATCCTGCAGTGGTCCTCTGAGCACGGGGA CAGAAAACACACGCGCTTTGCGGGGCCCTGACTCCCTTGGGT TTGACGTAAGGGTGGTTCAGTAACCC | 2119817 | 2119764 | 2119873 | 5 |
| 18_22 52278 | ATATAAAGAGTTTCCTTGGTTTTCATGCTGGCAGTGCCAGGGC ACCAAATCCCTCAGAGCTCGTCAACCAGCCCGGCTGCACTCC ACGCTGGCTGTGACTTTACAGATAG | 2252278 | 2252251 | 2252360 | 5 |
| 18_25 14413 | CCAGCCCTGCCCACACCCAGGTCTTAGATGTCCAGCTTCCAG GACTGAGAGAGACTCTGTTTCTGCTGCTTAAGCTGCCCTGGG AAACTAACGCAGAACAAGTAAATAAA | 2514413 | 2514331 | 2514440 | 6 |
| 18_27 24065 | GAAACCAGGCCTGCTCGCCCCCACGGTTAAGGCTACTCGGCT TTGAGACAACCAGGCTGAAATCACCTGTGTTTTGTTGGTGCT CCGTCTGCCAAGCAGCGAAAGCCTTC | 2724065 | 2724038 | 2724147 | 6 |
| 18_28 16068 | TATATGGCAACCAAAAACATGGCAGGGCGATGAATGGGAGTG GGTGGTCGTTACAGCTGGTGATGAGCGTATTTTAGTTCATTGT TCTAGTCTCTGGACTTTGGTGTCAG | 2816068 | 2816015 | 2816124 | 7 |
| 18_35 94827 | CATCAGAGAAAGGAGATTAAAATGACAATGAGATCCCATTAC CCACCCACCAATCTGTCAAAAATGGGGGAGGGGAGCTGCTG GCCTCCACACTGCTGATGCGCGTGTAA | 3594827 | 3594774 | 3594883 | 6 |
| 18_38 17775 | CCTTGTCGCTGAAGGGCAACGCCACTGTTTCTCTGACTCTCT CTGCAGCCAACTGGTGGTGGGAGCTGCACAGAGGCTTGTTTA CTGCTGGGGGCAGAGGGGGATGCAGA | 3817775 | 3817748 | 3817857 | 7 |
| 18_38 64387 | ACAGTCATTTTGGTTTCTCCTTGAGCCTGGCTCGTCGGGATGG TGAGTCTGGAAGGCACCCAGACCCCATGGCTGAGGGCGGAG GGCATGCACGGAGTTGGGTCTTTGAA | 3864387 | 3864360 | 3864469 | 5 |
| 18_39 63219 | AGGCAGTGCCCCTCTTAGTAAAGATGACACCTAAAGGTGCTT CCCTGAGTCCAAGCAGGTGATGTGCTGGGTGACTGAGAAGCT GGGGTATTAACCACCATCTATTTTTC | 3963219 | 3963166 | 3963275 | 6 |
| 18_39 88979 | TAAGATGAATGACCCAGGGGTCAGTGCCGAATTAGGGAAGGA TAAACCCTTCCGTGCCTCATCCTCTTCCCTGTACACCCAGAGT CCGTGGCATTCGGATGAGGAAGTCC | 3988979 | 3988897 | 3989006 | 6 |
| 18_43 17527 | CACAGGCTGATGCCCACACGAGGGTCTCAATGGGCCATGGGA ACAGATGCAATGCCGTGCAAACATTTCCAGCTGGGTTGTTGG CAGCCCGTGACTCAGGGTCCCCATCT | 4317527 | 4317474 | 4317583 | 6 |
| 18_46 06302 | TGTCTGGCACTTTCCTTCTCCCAGGGCGGCTGCGGGCAGGAT CAGAGCTTCGAGGCAGCCATTCTGGGCTCTTGTTGCATCATTT ATCATGAAAACGAGGCATTCGAATT | 4606302 | 4606275 | 4606384 | 5 |
| 18_55 34441 | CCTGGGAACCTCCGTATGTCGCACCTGTGGCCCTGAAAGAAA AACAAACATACAAACGATGAAGTCAGCGTGACATCACCCATC TCTGACACCGGAAGTACTCTAGGGTT | 5534441 | 5534359 | 5534468 | 6 |
| 18_56 | ATGAGGGGCCAGAGGAAGGGCTGGCAGCCTGATCGCACACG | 5630257 | 5630230 | 5630339 | 7 |
| 30257 | GAGCAGCTGGGCTCGCAAAATCCAAGCTCCTCAAGGTCTGCC TGGGCCGCTTCTCCCTTGCCCACCGTT | | | | |
| 18_63 01770 | AGCTGTCCTCCTGCCATACTCTATCTTCCACATGGTACTCAGAT GTGATGGCTGGAGCTCCAGCAGTCACTTTGGACTAGGAAGCC CAGGTCCATATCCTAGGTGGCTGC | 6301770 | 6301688 | 6301797 | 6 |
| 18_71 10407 | GCCCCTCATTTGCTGTGGGTCTTAGGGCCCCCGCTTTCCCTTT CGGCGAGAACGGCCCCTCCCTCCTCTGAGTCTTTGTCTCACC CTCTTCATGGACAAGGAGAACCCAT | 7110407 | 7110325 | 7110434 | 6 |
| 18_71 10407 | CCCCTCCCTCCTCTGAGTCTTTGTCTCACCCTCTTCATGGACA AGGAGAACCCATACCCTCTCCTCAGGAAAGCTTCTTGGAGAG AACACAGCTCTAACATTTCTGGATC | 7110407 | 7110380 | 7110489 | 5 |
| 18_76 34894 | AGAGATTGGCTATGCCTGGGGTTTTAGGCATAAACTAAGCAA CAGCCCTGCAGATATTAGCATCTTGATTTGTTCAAGGAATACT CCTGGAACCATAGCTAGGCGCAGGC | 7634894 | 7634812 | 7634921 | 5 |
| 18_76 34894 | ATTAGCATCTTGATTTGTTCAAGGAATACTCCTGGAACCATAG CTAGGCGCAGGCACAAAGCTTTGACATGTTCACCCCCAGAAT TCTATTGGGGTAAAGAAGGAGAGTG | 7634894 | 7634867 | 7634976 | 5 |
| 18_77 42000 | TAAGAGCTTACAATTGTTACACGCTTTTGACTAATCAGGCATT GGTCCAAGTTCTGCAGATGGTAAACCCCTCCGCATCGGCACG AGGCAGATGATGATTAGCCCATTCT | 7742000 | 7741973 | 7742082 | 5 |
| 18_82 45730 | TGCATGGCTTCACAGTTCTGGAGTCTAGAAGTCTAAAACCAA GGTGTAAGCAGGGCTCTGAGAGAGAACCTGTTCCTTGCTTCT GGCAGTCTTTGCTGTTCTTGGCTTGT | 8245730 | 8245648 | 8245757 | 6 |
| 18_82 45730 | CTCTGAGAGAGAACCTGTTCCTTGCTTCTGGCAGTCTTTGCT GTTCTTGGCTTGTGGATGTATCTCTGATCTCTGCTGCCTTCACT CTCACACGGTGTTAGCCTGTGTCT | 8245730 | 8245703 | 8245812 | 6 |

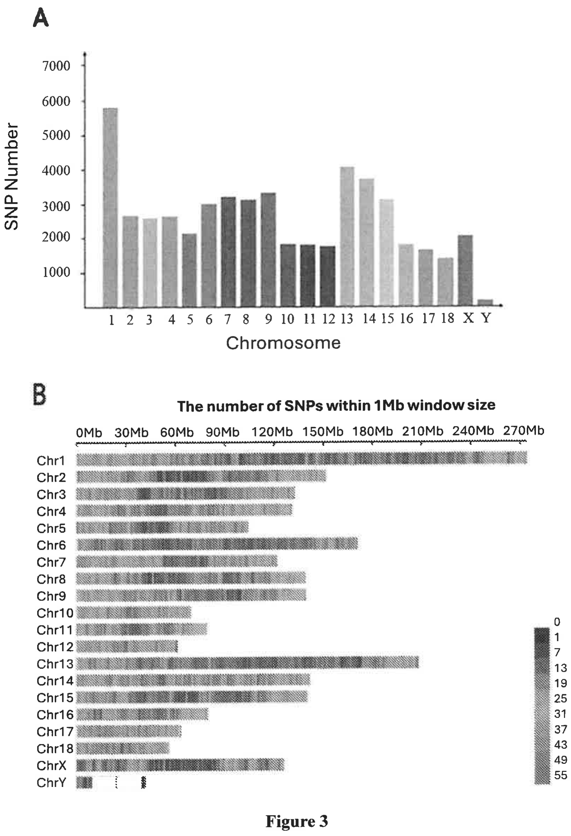

Compared to the 50K SNP solid-phase chip on the market, the number of target site SNPs provided by this invention is 52,000 (Figure 3). A total of 80,631 probes were designed for the target sites, and the number of detected SNP markers (mSNP, including target sites) was significantly increased to 80,000-100,000, providing more genomic information.

### Example 2: 50K mSNP Liquid-Phase Chip Preparation

This example demonstrates the preparation process of the 50K mSNP liquid-phase chip of the present invention. The specific steps are as follows:

### Step 1: Probe Preparation

Mix the synthesized pig 50K probes in equimolar amounts. Use EDTA and Tris-HCl (TE buffer) to dissolve to 3 pmol/mL, and prepare a pig 50K probe mixture for subsequent sequencing and hybridization.

### 1. Preparation of TE buffer

### 1×TE Buffer

Component concentration: 10mM Tris-HCl, 1mM EDTA, pH=8.0

### Preparation volume: 500 mL

Preparation method: Measure the following solutions into a 500 mL beaker:
1M Tris-HCl Buffer, pH=8.0, 5ml; 0.5M EDTA, pH=8.0, 1ml

Add about 400ml dd H₂O to the beaker, mix well; then dilute the solution to 500ml, and sterilize at high temperature and pressure; store at room temperature.

2. Use the prepared TE buffer to dissolve the pig 50K probes and prepare a 3 pmol/mL pig 50K probe mixture for subsequent sequencing and hybridization.

### Step 2: Preparation of probe hybridization solution

1. According to the library type, mix the following reagents in a 1.5mL PCR tube:

| Component name | Quantity |
|---|---|
| Pooled, barcoded library | 0.6 µL |
| GenoBaits Block I | 5 µL |
| GenoBaits Block II forILM/MGI | 2 µL |
| Invention probe | 300 ng |

2. Use a vacuum concentrator at a temperature of ≤60°C to concentrate to dryness;
3. After concentration, centrifuge at 12,000 rpm for 1 min. The prepared probes can be stored overnight at room temperature (15-25°C) for subsequent DNA library hybridization capture.

### Example 3: Use and Detection Method of the 50K Liquid-Phase Chip

This example demonstrates the operational process for using the 50K liquid-phase chip of the invention for genotyping. The specific steps are as follows:

### Step 1: Obtaining and extracting genomic DNA from the pig sample;

Select three common commercial pig breeds: Duroc, Large White, and Landrace, with 20 samples from each breed, totaling 60 samples. Extract genomic DNA from ear tissue. The specific method is as follows:
1. Shred the appropriate amount of ethanol-dehydrated pig ear tissue and place it in a 96-well deep plate (use a 2.0mL centrifuge tube if the amount is small). Add a 5mm steel bead, freeze with liquid nitrogen, and grind with a grinder for 1-2 minutes.
2. Add 500 µL Buffer PL2 and 5 µL Proteinase K (the diluted Proteinase K currently used in the lab) to the deep-well plate. Secure the cap and mix well using a shaker.
3. Incubate at 65°C for 30 min, periodically invert the plate for mixing during incubation.
4. Add 500 µL phenol-chloroform-isoamyl alcohol to the deep-well plate, mix well by shaking or pipetting up and down, and let it stand for 5 min.
5. Centrifuge at 4000 rpm for 10 min, transfer 400 µL of the supernatant to a new 96-well deep-well plate. (Ensure not to aspirate the middle sediment).
6. Add 800 µL PW solution and mix well.
7. Transfer the supernatant from step 6 to a 96-well centrifuge column in two batches, and perform vacuum filtration.
8. Add 600 µL WB I to the 96-well centrifuge column, incubate at room temperature for 2 min, and perform vacuum filtration. (Ensure anhydrous ethanol has been added to WB I as specified on the bottle).
9. Add 600 µL WB II to the 96-well centrifuge column and perform vacuum filtration. (Ensure anhydrous ethanol has been added to WB II as specified on the bottle).
10. Add 600 µL WB II to the 96-well centrifuge column and perform vacuum filtration.
11. Place the 96-well centrifuge column into an empty collection plate, centrifuge at 4000 rpm for 5 min. Place the 96-well centrifuge column on a new 96-well PCR plate and air dry at room temperature.
12. Add 60-100 µL preheated 65°C TE to the 96-well centrifuge column, incubate at room temperature for 2 min, and centrifuge at 4000 rpm for 5 min (preheating the TE to 65°C helps improve DNA elution efficiency and gel electrophoresis detection of target fragment length).

### Step 2: Constructing a pig cDNA library;

The specific steps include:
1. Probe Mixture
a) In a PCR tube, prepare the following reaction using the reagents of the present invention:

| | |
|---|---|
| DNA (lng-200ng) | _ µL |
| NucLease-free water | _µL |
| GenoBaits End Repair Buffer | 4 µL |
| GenoBaits End Repair Enzyme | 3.1 µL |
| Total | 20 L |

b) After gently mixing the reaction system, briefly centrifuge to collect the reaction liquid at the bottom of the tube.
c) Place the reaction tube in a PCR instrument for the following reaction at 82°C with the hot lid on:

| | |
|---|---|
| 37°C | 20 min |
| 72°C | 20 min |
| HoLd at | 4°C |

2. Adaptor ligation
a) Directly add the following components to the reaction system from Step 1:

| | |
|---|---|
| GenoBaitsULtra DNA Ligase | 2 µL |
| GenoBaitsULtra DNA Ligase Buffer | 8µL |
| GenoBaits Adapter for MGI | 2 µL |
| NucLease-free water | 8 µL |
| TotaL | 20 L |

b) After gently mixing the reaction system, briefly centrifuge to collect the reaction liquid at the bottom of the tube.
Note: The system must be thoroughly mixed; otherwise, library construction may fail.
c) Place the reaction tube in a PCR instrument for the following reaction, and cancel the hot lid: incubate at 22°C for 60 minutes, and then store at 4°C for later use.
3. DNA Purification
a) Take out GenoPrep DNA Clean Beads in advance and equilibrate at room temperature for over 30 min; vortex to mix before use.
b) Add 48 µL GenoPrep DNA Clean Beads to the ligation system from step 2, mix by vortexing, avoiding bubbles as much as possible; let stand for 5 min, and then briefly centrifuge to collect the liquid at the bottom of the tube.
c) Place the tube on a magnetic rack for at least 3 min until the solution is clear; remove the supernatant.
d) Keep the PCR tube on the magnetic rack, add 100 µL of 80% ethanol. Incubate at room temperature for 30 seconds, remove the supernatant.
e) Keep the PCR tube on the magnetic rack, open the cap and air dry for 5 minutes until the ethanol evaporates completely.
f) Remove the PCR tube from the magnetic rack and resuspend the beads with the PCR system in Step 4.

4. Library Amplification
Starting Amount and Recommended Amplification Cycles

| Starting Amount | Recommended Amplification Cycles |
|---|---|
| 1 ng-10 ng | 8 |
| 10-100 ng | 6-8 |
| 100 ng and above | 6 |

a) Prepare the following reaction in a new tube:

| | |
|---|---|
| GenoBaits PCR Master Mix | 10 µL |
| I5 Barcode (10µm) -MGI | 1 µL |
| I7 Barcode (2µm) -MGI | 5 µL |
| NucLease-free water | 4 µL |
| TotaL | 20 µL |

b) Add the above system to the beads dried in step 3, resuspend the beads, and briefly centrifuge to collect the reaction liquid at the bottom of the tube.
c) Place the reaction tube in the PCR instrument for the following reaction:

| | | |
|---|---|---|
| 98°C | 2 min | |
| 98°C | 30 s | 6-8 cycles. |
| 50°C | 30 s | |
| 72°C | 40 s | |
| 72°C | 4 min | |

5: Purification
a) Add 20 µL GenoPrep DNA Clean Beads to the system from step 4, mix by vortexing, avoiding bubbles as much as possible; let stand for 5 min, and then briefly centrifuge to collect the liquid at the bottom of the tube.
b) Place the tube on a magnetic rack for at least 3 min until the solution is clear; remove the supernatant.
c) Keep the PCR tube on the magnetic rack, add 100 µL of 80% ethanol. Incubate at room temperature for 30 seconds, remove the supernatant.
d) Keep the PCR tube on the magnetic rack, and air-dry with the cap open for 10 minutes.
e) Remove the PCR tube from the magnetic rack, add 35 µL Tris-HCl, vortex to mix, let stand for 5 min, and then briefly centrifuge to collect the liquid at the bottom of the tube.
f) On the magnetic rack, wait until the solution clears (about 3 minutes), and transfer the supernatant to a new tube, store at -20 °C.
g) The library requires further quality testing (e.g., concentration measurement and distribution assessment) for subsequent sequencing or the next step of the experiment.

### Step 3: Hybridization of pig genomic fragments with the probe of this invention, PCR of the samples, and purification, followed by sequencing;

The steps are as follows:
1. Use the mixed probe of this invention, melt at room temperature (15-25°C), mix well, and briefly centrifuge.
2. GenoBaitsBlock II, GenoBaitsBlock
a) According to the library type, mix the following reagents in a 1.5mL PCR tube:

| Component name | Quantity |
|---|---|
| Pooled, barcoded Library | 0.6-1µg |
| GenoBaitsBlock I | 5 µg (5 µL) |
| GenoBaitsBlock II for ILM/MGI | 2 µL |
| Invention probe | 300 ng |

b) Use a vacuum concentrator at a temperature of ≤60°C to concentrate to dryness;
c) After concentration is complete, centrifuge at 12000 rpm for 1 min, and then proceed with subsequent operations.
3. Hybridization capture of the DNA library
a) Dissolve all GenoBaits hybridization reagents at room temperature;
b) Add the reagents to the tube;
c) Pipette or vortex to mix well, centrifuge at 12000 rpm for 1 min, let stand at room temperature for 5 min, pipette or vortex to mix again, lightly centrifuge, and transfer the entire mix to a 0.2 mL EP tube;
d) Thermal cycling incubation conditions: 95°C for 10 min (lid temperature at 105°C);
e) Once the PCR cycler cools down to 65°C, transfer it to another PCR machine with a lid temperature of 75°C and 65°C for hybridization. Note: If necessary, the experiment can be conducted overnight at 65°C (14-16h). *Hybridization at 65°C helps improve capture efficiency.

4. Preparation of elution buffer (Wash Buffer)
Single capture system, dilute GenoBaits buffers to 1 × system.
5. Preparation of GenoBaits DNA Probe Beads
a) Place GenoBaits Probe Beads at room temperature for 10 min before use;
b) Vortex for 15 seconds to mix well;
c) Prepare 50 µL of GenoBaits Probe Beads for each reaction, place them in a 0.2 mL EP tube;
d) Place the tube on a magnetic rack, allowing the beads to fully separate from the solution.
e) Remove the supernatant, retain the beads
f) Elution: For each reaction, add 150 µL of GenoBaits 1X Bead Wash Buffer, vortex for 10 seconds, transfer the tube to the magnetic rack, let the beads fully separate from the solution, and remove the supernatant.
g) Repeat step 6 above twice for a total of three washes.

6. Binding of hybridized fragments with GenoBaits DNA Probe Beads
a) Transfer the entire 16 µL of hybridization solution to the prepared beads
b) Vortex for 10 seconds to mix well, centrifuge for 5 seconds.
c) Place the EP tube in the PCR machine at 65°C for 45 minutes, with a heat cover temperature of 75°C (to bind DNA with the beads)
d) Shake for 5 s every 12 min.

7. Elution to remove unbound DNA (using 1× Wash Buffer from step 4)
a) Prepare a 65°C elution buffer (completed on the PCR machine)
b) Prepare a room temperature elution buffer
c) Resuspend the beads, the suspension is used for step 8, and keep the remaining 10µL as a backup.

8. PCR enrichment
a) According to the library type, prepare PCR reagents in a 0.2 mL PCR tube
b) Briefly vortex, centrifuge, and ensure the beads are still in the solution
c) Place the PCR tube in the PCR machine, with the heat cover temperature at 105°C, for PCR amplification

9. PCR Product Purification
a) Add 45 µL (1.5X volume) GenoPrep DNA Clean Beads to each PCR reaction, mix by vortexing, avoiding bubbles as much as possible; let stand for 5 min, and then briefly centrifuge to collect the liquid at the bottom of the tube.
b) Place the tube on a magnetic rack for at least 3 min until the solution is clear; remove the supernatant.
c) Keep the PCR tube on the magnetic rack, add 100 µL of 80% ethanol. Incubate at room temperature for 30 seconds, remove the supernatant.
d) Keep the PCR tube on the magnetic rack, and air-dry with the cap open for 10 minutes.
e) Remove the PCR tube from the magnetic rack, add 35 µL Tris-HCl, vortex to mix, let stand for 5 min, and then briefly centrifuge to collect the liquid at the bottom of the tube.
f) On the magnetic rack, wait until the solution clears (about 3 minutes), and transfer the supernatant to a new tube, store at -20 °C.
g) The library requires further quality testing (e.g., concentration measurement and distribution assessment) for subsequent sequencing or the next step of the experiment.

10. Library testing
a) Measure the library with Qubit Fluorometer and Qubit dsDNA HS Assay Kit
b) Measure the average length of captured DNA library fragments on a digital electrophoresis system
c) Measure the library concentration with a KAPA Library Quantification Kit

11. Sequencing
Operate according to the requirements of the sequencing instrument. The average sequencing depth for target site SNPs is 105.88X.

### Step 4: mSNP Genotyping

Genotypes for all mSNP sites are obtained according to the sequencing data processing workflow. The specific steps are as follows:
1. Use Trimmomatic software to remove adapters and low-quality reads.
2. Use BWA software to align the reads of each individual to the pig reference genome Sscrofa11.1 (GCA_000003025.6);
3. Use SAMtools to generate BAM and sorted BAM files;
4. Use the GATK pipeline to generate a VCF file containing all mSNPs (including target site SNPs).

Example 3 demonstrates the operational process for using the liquid-phase chip of this invention, while Example 4 evaluates the genotyping quality of the liquid-phase chip in samples from multiple pig farms.

### Example 4: Evaluation of Genotyping Quality of 50K mSNP Liquid-Phase Chip

Blood samples were collected from Duroc, Landrace, and Large White pigs from multiple farms. Genotyping was performed using this invention as described in Example 3, to evaluate the stability of the detection and the quality of mSNP markers.

### 1. Stability

The stability of chip detection was generally measured by the consistency and correlation coefficient of the genotyping results from two tests of the same repeated sample. The genotyping consistency (0.992 (0.001)) and correlation coefficient (0.996 (0.001)) of the 60 repeated samples from the Pig 50K mSNP liquid-phase chip were both greater than 99%, indicating good genotyping stability.

### 2. mSNP Quantity and Quality in Different Pig Populations

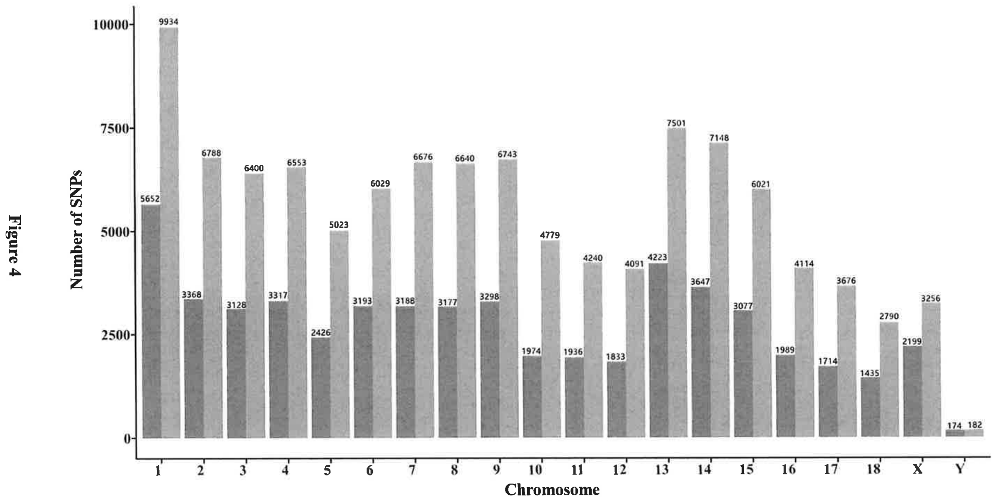

The Pig 50K mSNP liquid-phase chip developed by this invention has 52,000 target sites. After preliminary filtering of the sequencing data, the target sites were all detected as shown in Table 2. However, due to population differences (some populations had non-polymorphic mSNP sites), the number of detected mSNPs varied somewhat, as shown in Table 2. The three pig farms detected 52,000 target sites, with a total of 108,559-108,585 mSNPs detected, with slight differences but no significant variation. This indicates that the SNP sites designed by this invention are universally applicable across different farms and can be widely used in practical populations. Additionally, as shown in Figure 4, the number of mSNPs on each chromosome increased significantly.

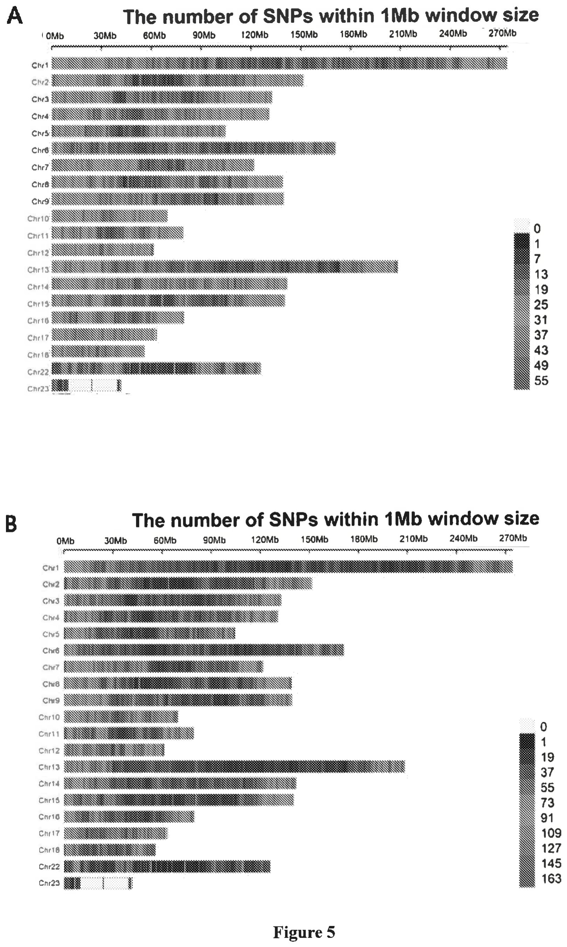

The density distribution of chromosomes is similar between the two, and the increase in mSNPs enhanced the SNP density without changing the general distribution of SNPs (Figure 5), as shown in other pig farms as well. This indicates that in practical applications, the detection of mSNPs is consistent with the characteristics of multiple single nucleotide polymorphism detection technology, demonstrating that the mSNP detection technology of the present invention can effectively amplify the number of SNP markers, thereby improving the efficiency of fragment capture.

Moreover, the mSNP liquid-phase chip shows almost no difference between mSNPs (including target sites) and target sites in terms of missing rate and MAF, indicating that the amplification of SNP numbers by the mSNP liquid-phase chip does not reduce the quality of the chip data.

**Table 2 shows the number of target sites and mSNPs before and after quality control for the 50K mSNP liquid-phase chip in three pig farms.**

| Pig Farm | Number of Samples | Original Number of Target Sites | Original Number of Markers | Number of Target Sites After Quality Control | Number of Markers After Quality Control |
|---|---|---|---|---|---|
| Farm 1 | 534 | 52000 | 108585 | 43943 | 81461 |
| Farm 2 | 42 | 52000 | 108564 | 43998 | 89851 |
| Farm 3 | 97 | 52000 | 108559 | 43136 | 89167 |

### 3. Post-Quality Control Status of Genotypes in Different Populations

Genotype quality control (referred to as 'QC') is a routine operation after chip detection to ensure the quality of downstream analysis and is largely influenced by the population. In this example, the following QC steps were applied to multiple pig populations using this invention:
Remove sites with unknown positions; remove SNPs with a call rate lower than 90%; remove SNPs with a minor allele frequency (MAF) lower than 0.05; remove SNPs with a significant deviation from Hardy-Weinberg equilibrium (P<10⁻⁶).

As shown in Table 2, a small number of SNPs were deleted after quality control for the 50K mSNP liquid-phase chip in the three pig farms. After quality control, there were 81,461 to 89,851 mSNP markers remaining, including 43,136 to 43,998 target sites. If the target sites did not meet the quality control standards, the mSNPs within the probe would also not meet the quality control criteria. After quality control, the number of mSNP markers did not decrease significantly, remaining approximately twice the number of target sites; this indicates that this invention has selected high-quality SNPs upstream and downstream of the target sites, thereby increasing genomic information.

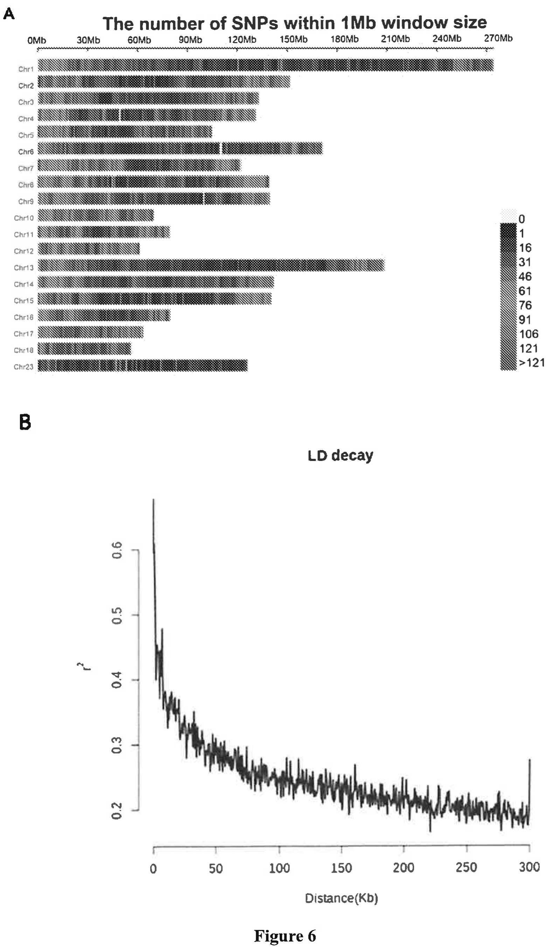

Taking Farm 1 as an example, as shown in Figure 6, the data distribution after quality control of the mSNP liquid-phase chip did not change significantly, with the LD decay trend normal, but the average linkage disequilibrium (r2 = 0.45) was higher compared to using only target sites (r2 = 0.2), helping to improve the efficiency of genetic analysis and genomic selection. This indicates that after quality control, the mSNP can significantly increase the number of SNP detections without reducing data quality, which helps to retain more effective variations, thereby improving the efficiency of variation detection.

Example 4 evaluates the high stability and good genotype quality of this invention, making the liquid-phase chip suitable for whole-genome association analysis and genomic selection. Example 5 takes genomic selection as an example to demonstrate the application effects of this invention.

### Example 5: Application of the 50K mSNP Liquid-Phase Chip in Genomic Selection

After sampling 800 Large White pigs with growth and reproduction data, genotyping was performed using this invention for genomic selection. These individuals also have genotype data from the solid-phase chip SNP chip Porcine GGP 50K (referred to as GGP50K, Neogen Corporation, USA).

### 1. Genotype Detection and Quality Control

Genotype quality control is an essential means to ensure the rationality of subsequent genetic analysis and molecular breeding results after genotyping is completed for all chips (including this invention). In this example, the following quality control steps were applied sequentially:
1) Filter out multi-allelic SNPs; 2) Remove sites on sex chromosomes and sites with unknown positions; 3) Remove SNPs with a call rate lower than 90%; 4) Remove SNPs with a minor allele frequency (MAF) lower than 0.05; 5) Remove individuals with a call rate lower than 90%.
   After quality control, all individuals were retained, and 88,105 mSNP sites of this invention were retained, including 42,302 target site SNPs. The GGP50K chip retained 41,296 SNPs. The number of SNP markers on the GGP50K chip after quality control is close to the number of target site SNPs of this invention.
2. Comparison of Genomic Selection Accuracy between This Invention and GGP50K Table 3 shows the comparison of genomic selection effects between this invention and the mainstream chip GGP50K, with a similar number of markers on both chips. After quality control of genotypes, the 50K liquid-phase chip had 88,105 mSNP markers, including 42,302 target site SNPs, close to the number of 41,296 SNPs after GGP50K quality control. However, the genomic selection accuracy of GGP50K for three traits was lower than that of this invention, with genomic selection accuracy lower by 1%-4% when using all mSNP markers (88,105) and lower by 1.8%-5.4% when using only target sites (42,302). The results indicate that the selection of target sites in this invention ensures better genomic selection effects than GGP50K based on solid-phase chip technology. On the other hand, unlike solid-phase chips, the mSNP liquid-phase chip increased markers through multiple single nucleotide polymorphism technology. However, traditional single-marker analysis methods did not show the advantage of marker increase, with genomic selection accuracy slightly lower than using only 42,302 target sites.

It should be noted that genomic selection is currently the main method of molecular breeding, and its application effect is mainly measured by genomic selection accuracy. The accuracy of genomic selection for most traits is mainly improved by expanding the reference population (with both phenotypic and genotypic data) and evaluation methods. Expanding the reference population by one time means doubling the breeding cost, with accuracy only improving by 5-9%, and the improvement for some traits is even more challenging. The invention, under the same population size, has achieved an improvement in accuracy of the liquid-phase chip over GGP50K in some traits, equivalent to the effect of doubling the population size.

**Table 3 Comparison of Genomic Selection Accuracy between This Invention and Solid-Phase Chips**

| SNP Type | Number of Markers | Days to Reach 100kg Body Weight (AGE) | 100 kg Live Backfat Thickness (BF) | Total Number of Piglets Born (TNB) |
|---|---|---|---|---|
| GGP50K | 41296 | 0.514 | 0.589 | 0.542 |
| Target sites | 42302 | 0.562 | 0.607 | 0.596 |
| mSNPs | 88105 | 0.554 | 0.599 | 0.588 |

Although Example 5 demonstrates that this invention can be used for genomic selection and has advantages over solid-phase chips, the traditional single-marker analysis methods did not show the advantage of increasing the number of mSNP markers. This invention proposes an improved mSNP analysis method, and Example 6 demonstrates its application in genomic selection. Likewise, the new method can also be used in whole-genome association analysis and other genetic analyses.

### Example 6: Application of the New mSNP Analysis Method of This Invention in Genomic Selection

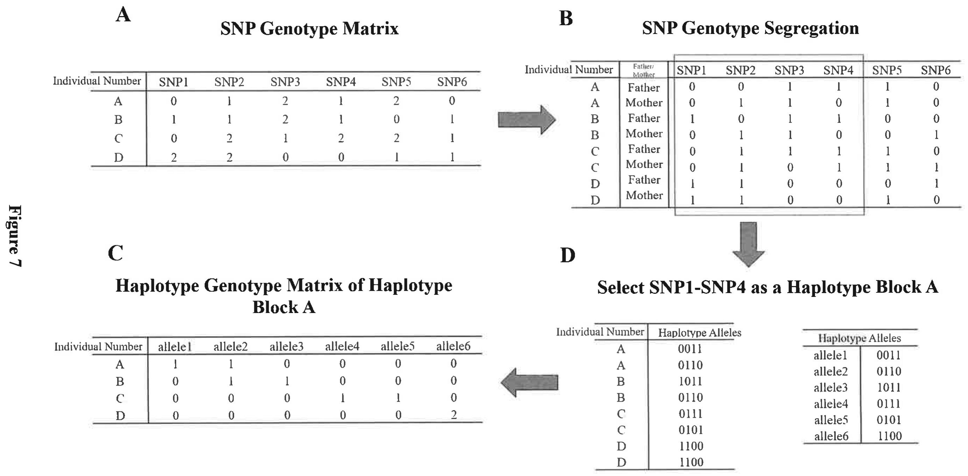

Example 5 demonstrates that although the mSNP liquid-phase chip increases the number of mSNP markers and has higher genomic selection accuracy than the GGP50K designed by Neogen in the United States, it does not show a marker quantity advantage compared to genomic selection using only target site SNPs. This is mainly due to the limitations of traditional analysis methods. Therefore, this invention proposes an mSNP utilization strategy based on haplotype analysis. This example illustrates the advantages of the new analysis method of this invention.
1. The pig population, phenotypic data, and genotypic data are the same as in Example 5.
2. The genotype quality control standards and the number of individuals and markers after quality control are the same as in Example 5.
3. Haplotype block partitioning: This invention proposes a haplotype block partitioning strategy centered on target site SNPs. With the target site SNPs of the 50K liquid-phase chip prepared by this invention as the center, the mSNPs within 200bp upstream and downstream of the target sites are used as a haplotype block to construct haplotypes (named targeting block), and the number of targeting blocks is the same as the number of target sites after quality control.
4. Haplotype allele and genotype matrix construction
   Within each targeting block, a haplotype matrix is constructed for all tested samples. As shown in Figure 7, within each haplotype block (i.e., targeting block), individual haplotypes are re-encoded. In Figure 7A, a genotype matrix for 4 individuals with 6 SNP markers is shown, where 0, 1, and 2 represent homozygous, heterozygous, and the other homozygous genotype, respectively. The four SNPs indicated by the red box B in Figure 7 form a targeted block. First, haplotype inference is performed based on SNP genotypes to obtain the paternal and maternal haplotypes for each individual within each haplotype block. After classifying all haplotypes, they are encoded as alleles. Then, for each haplotype allele within the haplotype block, individual diplotypes are encoded as 0, 1, or 2, representing the number of copies of a particular haplotype allele carried by the individual. As shown in Figure 7C, after haplotype inference of the four SNPs, there are six haplotypes serving as alleles. Finally, an N-H matrix is generated, where N is the number of individuals, and H is the total number of haplotype alleles, as shown in Figure 7D. After re-encoding the haplotypes of the 4 individuals according to the haplotype alleles, a 4*6 haplotype matrix is generated, where 4 represents the number of individuals, and 6 represents the number of haplotype alleles.
5. Genomic Selection Accuracy of the New mSNP Analysis Method of This Invention Table 4 shows the effect of genomic selection for pig growth and reproductive traits using the new mSNP analysis method of this invention. We also compared the effectiveness of the targeted block with three other haplotype block partitioning methods. The 2 SNPs/block and 5 SNPs/block methods set 2 and 5 SNPs, respectively, as the size of the haplotype blocks, without overlap, for haplotype construction. 400bp/block is the partitioning of blocks with a fixed 400bp physical distance, non-overlapping, for haplotype construction. Among the four haplotype block partitioning methods, the targeted block proposed in the present invention achieves the highest genomic selection accuracy. Although the 400 bp/block method yields results similar to those of the targeted block, it requires traversing the entire genome, resulting in excessive computation time. In contrast, the targeted block selectively focuses on mSNPs near the target loci, significantly reducing computation time.

**Table 4 Advantages of the New mSNP Analysis Method Proposed by This Invention for Genomic Selection**

| SNP/Haplotype | Number of Haplotype Alleles or SNP Markers | Days to Reach 100kg Body Weight (AGE) | 100 kg Live Backfat Thickness (BF) | Total Number of Piglets Born (TNB) |
|---|---|---|---|---|
| Target sites | 42302 | 0.562 | 0.607 | 0.596 |
| 2 SNPs/block | 153367 | 0.573 | 0.608 | 0.622 |
| 5 SNPs/block | 210348 | 0.567 | 0.604 | 0.616 |
| 400bp/block | 238449 | 0.599 | 0.629 | 0.642 |
| Targeting block | 240015 | 0.599 | 0.629 | 0.643 |

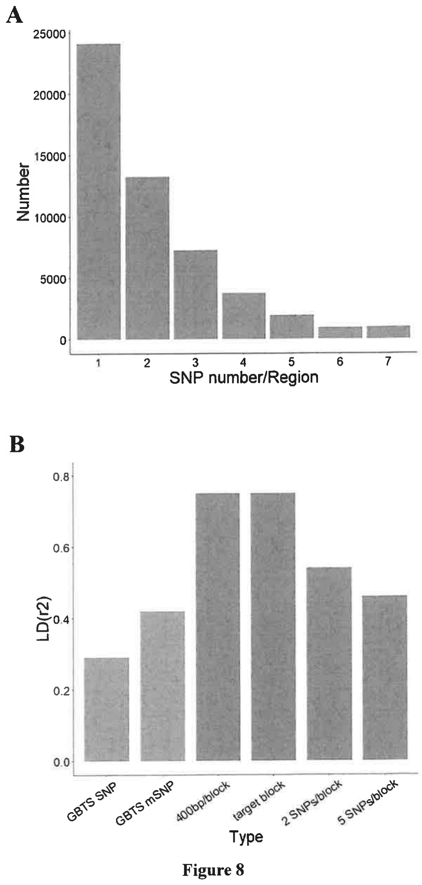

After quality control, the mSNP chip had 88,105 mSNP markers, including 42,302, forming 42,302 targeting haplotype blocks. As shown in Figure 8A, 50% of the blocks contained more than 2 mSNPs (including target sites), adding 45,803 markers, increasing the number of markers. As shown in Figure 8B, these mSNPs are in high linkage disequilibrium with the target loci (r²=0.75), but not in complete linkage disequilibrium. Therefore, they exhibit haplotype polymorphism and can provide more information than a single SNP (the average linkage disequilibrium between adjacent target loci is 0.3), resulting in higher genomic selection accuracy compared to using only the target loci. These applications are consistent with the design of this invention, selecting mSNPs with medium to high linkage disequilibrium. Therefore, the mSNP liquid-phase chip can improve genomic selection accuracy through the haplotype analysis strategy proposed by this invention without increasing application costs, that is, the new mSNP analysis method proposed by this invention has the best genomic selection effect. This can also be extended to whole-genome association analysis and other genetic analyses.

## Claims

1. A method for SNP marker selection and probe preparation for a 50K liquid phase chip used for multiple single nucleotide-polymorphism, which utilizes whole-genome sequencing data of pig breeds to mine and screen target SNP loci, then designs and optimizes probes for the target SNP loci, ultimately resulting in the determination of the probes. The method includes the following steps:
Step 1, determining target site SNPs: Based on whole-genome sequencing data of three pig breeds-Duroc, Large White, and Landrace-and aligning them to the whole-genome sequencing data of pig breeds, genomic regions with moderate linkage disequilibrium between markers were selected, and targeted capture sites were screened, i.e., target site SNPs.
Step 2, designing probes based on the determined target site SNPs: Utilize the multiple single nucleotide polymorphism technologies to design 1-4 probes, each 110 bp in length, centered on each target SNP. Each probe covers the target SNP, with a total probe coverage of 165 bp around the target SNP. The principles for probe design are: 1) Select probes with a content between 30% and 80%; 2) Choose regions with a number of homologous areas ≤ 5; 3) Select probe areas that do not contain SSR, N regions.
Step 3, selecting and optimizing probes containing high-quality mSNPs: Probes are hybridized and sequenced, and the genotyping quality of mSNPs, including target sites, is detected. Set a missing rate of NA<0.1, a minimum allele frequency (MAF) ≥0.05, and heterozygosity (Het) <0.5 as standards to screen mSNPs, removing those that do not meet the standards. If the probe does not meet the genotype quality control requirements of mSNPs, delete the probe and the corresponding target site SNP, redesign new probes according to Steps 1 and 2, and continue to test and optimize the probes as per this step. The mSNPs that meet the quality inspection requirements are finally used as the target sites of the 50K mSNP liquid-phase chip.

2. The method according to claim 1, wherein the whole-genome sequencing data of pig breeds is based on the 11.1 reference genome; the principles for selecting target SNPs are: ① Uniform distribution across chromosomes, with denser distribution at both ends of the chromosomes; ② Polymorphism considers MAF > 0.35 in Duroc, Landrace, and Large White pigs; ③ Average linkage disequilibrium (r2) with upstream and downstream SNP markers less than 0.85; ④ Comparison with the QTLdb database, aiming for SNP markers to be located in QTL regions related to economic traits; ⑤ overlap with some loci on the known 50K chip for pigs.

3. The method according to claim 2, wherein the known pig 50K chip is a 50K SNP liquid phase chip, GGP50K from Neogen Corporation, or Zhongxin No. 1.

4. A 50K mSNP liquid-phase chip for pigs based on multiple single nucleotide polymorphisms, **characterized in that** it is prepared from probes obtained by the method according to any of claims 1 to 3.

5. The pig 50K mSNP liquid phase chip according to claim 4, wherein after synthesizing the probes, they are mixed in equal molar amounts, diluted to 1-5 pmol/mL in the buffer solution, and then prepared into the probe hybridization solution.

6. The 50K mSNP liquid-phase chip for pigs according to claim 5, **characterized in that** the buffer solution is a mixture of EDTA and Tris-HCl.

7. The 50K mSNP liquid-phase chip for pigs according to claim 5, **characterized in that** it further includes using a Pooled, barcoded library, GenoBaits Block I, and GenoBaits Block II for ILM/MGI to prepare the probe hybridization solution with the following components:
| Component name | Quantity |
|---|---|
| Pooled, barcoded library | 0.6 µL |
| GenoBaits Block I | 5 µL |
| GenoBaits Block II for ILM/MGI | 2 µL |
| The said probe | 300 ng |

8. The 50K mSNP liquid-phase chip for pigs according to claim 7, **characterized in that** the probe hybridization solution is concentrated to dryness using a vacuum concentrator at a temperature ≤60°C.

9. A method for detecting the genotype of individual pigs using the 50K mSNP liquid-phase chip for pigs according to any of claims 4 to 8. The said method includes the following steps: obtaining samples from the pigs to be tested and extracting genomic DNA; constructing pig cDNA libraries; hybridizing and sequencing the constructed libraries with the pig 50K mSNP liquid-phase chip; performing mSNP genotyping according to the sequencing data operation process, and determining the genotypes of all liquid-phase chip marker loci for each individual.

10. The method according to Claim 9, **characterized in that** the mSNP genotype analysis method is as follows:
Step 1: After determining the genotypes of all mSNP markers for the individual liquid-phase chip, perform quality control on the mSNP genotypes; quality control is carried out in the following order:
1) Filter out multi-allelic variants;
2) Remove sex chromosomes and loci with unknown positions;
3) Remove SNPs with a call rate below 90%;
4) Remove SNPs with a minor allele frequency (MAF) below 0.05;
5) Remove individuals with a call rate below 90%.
Step 2: Using the target site SNP as the core, define a 200bp upstream and downstream region as a haplotype block, dividing the genome into 52,000 haplotype blocks, each with at least one mSNP marker, with varying numbers.
Step 3: For each haplotype block, infer haplotypes, determine haplotype alleles, and construct haplotype genotypes or diplotypes for each haplotype block in the tested sample, thereby constructing diplotype vectors for all haplotype blocks in the tested sample, similar to genotype vectors for all mSNP markers.
Step 4: Based on the diplotype vectors of all samples, apply genetic analysis or molecular breeding methods, with each haplotype block treated as a marker, and haplotypes within the block as alleles and diplotypes as genotypes.
